# EUROPEAN PATENT APPLICATION

(11) **EP 2 110 129 A1**
(43) Date of publication of application: **21.10.2009**
(21) Application number: 08305112.8
(22) Date of filing: 18.04.2008
(51) Int. Cl.: A61K 31/165, A61P 25/24

(54) **Use of enantiomer (1S, 2R) milnacipran hydrochloride for the preventive treatment of suicidal behaviour in depressed patients**

(71) Applicant: PIERRE FABRE MEDICAMENT, 92100 Boulogne-Billancourt (FR)
(72) Inventor: Mansuy, Lucilla, 31240, L'UNION (FR)
(74) Representative: Warcoin, Jacques

(57) **Abstract**

The invention concerns the substantially pure (1S, 2R) enantiomer of milnacipran hydrochloride for its use for treating patients suffering from psychiatric disorders, while reducing the risk of suicidal behaviour or ideation in said patients, and/or for limiting the risk of suicidal behaviour or ideation induced by the treatment with one or several SSRIs or other actual anti-depressants.

## Description

The present invention relates to the use of the substantially pure (1S, 2R) enantiomer of milnacipran, particularly to the use of the substantially pure (1S, 2R) enantiomer of milnacipran hydrochloride, F2695, for the preparation of a drug intended to treat patients suffering from psychiatric disorders, while limiting the risk of suicidal behaviour or ideation. More specifically, the enantiomer F2695 in accordance to the invention is intended to treat depression, such kind of disease being associated with a high risk of suicidality.

Globally, a million people commit suicide every year, and 10-20 million attempt it. Mood disorders, especially major depressive disorder (MDD) and bipolar disorders are the most common psychiatric conditions associated with suicide. Primary (psychiatric and physical illness), secondary (psychosocial), and tertiary (demographic) risk factors for suicide have been identified. Comorbid psychiatric illness, particularly anxiety symptoms or disorders, significantly increase the risk of suicidal behavior. Thus, to identify patients at risk, current standard risk assessments and precautions may be of limited value, while assessing the severity of anxiety and agitation may be more effective.

Once a patient is diagnosed for MDD, several treatments are possible, most of them relying on the use of antidepressants.

It has been suggested that the use of typical antidepressants such as Selective Serotonin Reuptake Inhibitors (SSRI) may increase the risk of suicide, possibly due to a combination of akathisia, emotional disinhibition, emotional blunting, manic or psychotic reactions mediated by the alleviation of the motor retardation of depression observed during the first phase of the treatment (Healy D, *PloS Medicine* 2006).

As a matter of fact, according to a report from the FDA (US Food and Drug Administration), pooled analyses of short-term (4 to 16 weeks) placebo-controlled trials of nine antidepressant drugs (SSRIs and others) in children and adolescents with major depressive disorders (MDD), obsessive compulsive disorders (OCD), or other psychiatric disorders have revealed a greater risk of adverse events representing suicidal thinking of behaviour (suicidality) during the first few months of treatment in those receiving one of these anti-depressants. Indeed, on a total of 24 trials involving over 4400 patients, the average risk of such events in patients receiving these anti-depressants was 4 %, twice the placebo risk of 2 % (FDA, February 3, 2005, see on http://www.fda.gov/cder/drug/infopage/paroxetine/default.htm:prescribing information pdf).

In front of the risk of treatment-related activation of suicidal behaviours, FDA issued a black box warning that all anti-depressants may increase the risk of suicidal thinking and behaviour in children and adolescents. This black box must be cited in all advertising as well as included in the package insert. In addition, a new medication guide must be distributed by pharmacists to all who obtain such medications. Close observation by therapists and families is then considered necessary to follow the patients treated with such medications.

Consequently, there was a sharp decrease in anti-depressant prescriptions for children (Gibbons RD, American Journal of Psychiatry 2007; 164: 1356-1363), with uncertain public health impact. In front of this, however, the American Medical association and American Psychiatric Association both warned against decreasing access to these drugs for patients who may benefit from them.

Consequently, there is an urgent need to find a substitute to antidepressants that does not stimulate suicidal behaviour or ideations, i.e. a medication that effectively treats psychiatric disorders without increasing the risk of suicidality. More importantly, there is an urgent need of a medication that actually reduces the risk of suicidality in patients suffering from psychiatric disorders, and in particular from depression, as it is known that the suicidal risk is a main problem in patients suffering from depression, probably more than in patients suffering from other psychiatric disorders.

This treatment would be particularly appropriate for depressed young patients such as children, adolescents, and young adults, which are more sensitive to suicidal behaviour than older patients. This treatment would also be intended to treat older patients that have suicidal behaviour or ideation.

Since today, lithium is the medication that has most consistently demonstrated an antisuicidal effect. However, three new atypical anti-psychotic drugs have also been described for such a use: Sertindole, which is an antipsychotic drug with high affinity for serotonin 5-HT₂, dopamine D₂ and α1 adrenergic receptors (US 2007/0281925), 5-HT2C receptor antagonists (WO 2004/082584), and the atypical antipsychotic drug clozapine, may limit suicidal behaviour in human subjects with schizophrenia or psychiatric disorders (Munro J et al, Brain Journal Psychiatry 1999, 175:576-580).

Concerned to find a medication that can be added to or substitute the SSRI in the treatment of psychiatric disorders, in particular depression, in order to limit or better to reduce the risk of suicidality, the inventors have now discovered that, surprisingly and unexpectedly, the (1S, 2R) enantiomer of the milnacipran hydrochloride, F2695, not only prevents the increase, but also is able to decrease the risk of suicidality associated with psychiatric disorders such as depression.

F2695 is the (1S, 2R) enantiomer of milnacipran hydrochloride, which corresponds to the dextrogyral enantiomer of milnacipran hydrochloride.

F2695 is the pharmacologically active enantiomer of milnacipran hydrochloride, as it is two and three times more efficient than the racemate in norepinephrine and in serotonine uptake respectively, as demonstrated on a homogenate of rat hypothalamus (US 7,005,452). Accordingly, F2695 is considered to be an active dual inhibitor of serotonin (5-HT) and norepinephrine (NE) reuptake.

F2695, chemically named Z-(1S, 2R)- 2-(amino methyl)-N,N-diethyl-1-phenyl cyclopropane carboxamide hydrochloride, has the following structure:

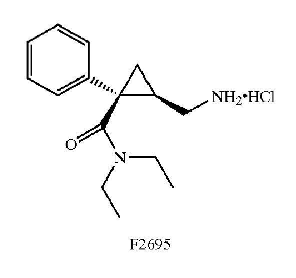

The enantiomer can be separated and isolated from the racemate using procedures described in the literature (Bonnaud et al, Journal of chromatography, vol 318: 398-403);

A method of manufacturing F2695 is described in Shuto et al, Tetrahedron letters, 1996 Vol 37: 641-644; and Doyle et Hu, 2001, Advanced Synthesis and Catalysis, Vol.343: 299-302.

Research directed at the therapeutic effectiveness of F2695 has focused on its use in the treatment of psychiatric disorders, particularly depression while avoiding cardiovascular disturbances and/or organ and/or tissue toxicity. F2695 may also be effective in the treatment of other disorders such as pain, drug addiction and urinary incontinence. It has also been shown that F2695 has a superior toxico-genomic profile and a significantly better safety coefficient than other psychotropic products such as clomipramine and milnacipran (US 7,005,452).

The object of the present invention is the substantially pure (1S, 2R) enantiomer of milnacipran hydrochloride, F2695, for its use for treating patients suffering from psychiatric disorders, while reducing the risk of suicidal behaviour or ideation in said patients.

Another object of the present invention is the use of the substantially pure (1S, 2R) enantiomer of milnacipran hydrochloride for the preparation of a drug intended to treat patients suffering from psychiatric disorders while reducing the risk of suicidal behaviour or ideation in said patients.

In the context of the present invention, "substantially pure (1S, 2R) enantiomer of milnacipran hydrochloride" is intended to mean a mixture of both (1S, 2R) and (1R, 2S) enantiomers of milnacipran hydrochloride containing more than 98% by weight of the (1S, 2R) enantiomer, preferably more than 99% by weight of the (1S, 2R) enantiomer, more preferably 100% by weight of the (1S, 2R) enantiomer.

For the purpose of the invention, "F2695" corresponds to the substantially pure (1S, 2R) enantiomer of milnacipran hydrochloride.

For the purpose of the invention, the phrases "reducing the risk of suicidal behaviour and ideation" or "reducing the risk of suicidality" is understood to mean the fact of reducing these risks significantly in a patient following administration of the drug.

As used herein, "suicidal behaviour and ideation" refers to possible suicide-related adverse events, as identified by the Columbia University group for the classification of the pediatric suicidality data: completed suicide, suicide attempt, preparatory acts towards imminent suicidal behaviour, and suicidal ideation. Suicidal behaviour comprises performing actions that put him / her at risk of death. Suicidal behaviour may include acts of self-harm with a fatal (suicide) or non-fatal (attempted suicide) outcome.

Also, as it is known that major depressive disorders are associated with an important risk of suicidality, and that this risk is also related to the severity of the depressive episode, the inventors/clinicists associate suicidal risk, consequently to clinical observation, to the HAM-D 17 score superior to 22 points.

One particular embodiment of the invention is to use the substantially pure (1S, 2R) enantiomer of milnacipran hydrochloride, F2695, for reducing the risk of suicidal behaviour or ideations, i.e. to administer a pharmaceutical composition comprising F2695 to a human subject who suffers from psychiatric disorders and has a suicidal behaviour or ideations, with the result that said suicidal behaviour is reduced, thereby leading to a lower frequency of suicide attempts or even to a complete prevention of the human subject committing or attempting to commit suicide.

An other embodiment of the invention is to administer the pharmaceutical composition in patients suffering from psychiatric disorders, preferably depression. In this case, the substantially pure (1S, 2R) enantiomer of milnacipran hydrochloride, F2695, in accordance with the invention is administered to all types of patients requiring such a treatment, whether it be for therapeutic and/or prophylactic purposes. For therapeutic purposes, the aim is to eradicate or to improve the condition to be treated (here psychiatric disorders) and one or more related symptoms (here, suicidal behaviour or ideation). For prophylactic purposes, the aim is to prevent the appearance of the condition to be treated (here psychiatric disorders) and one or more related symptoms (here, suicidal behaviour or ideation).

Nevertheless, the substantially pure (1S, 2R) enantiomer of milnacipran hydrochloride, F2695, in accordance with the invention is particularly adapted to populations of at-risk patients, in particular young patients (age 0-24) suffering from psychiatric disorders or patients that have suicidal behaviour or ideations.

Young patients are advantageously chosen among children, adolescents, and young adults (age 18-24), all of them suffering from psychiatric disorders.

Because of its pharmacological properties, in particular as dual inhibitor of serotonin (5-HT) and norepinephrine (NE) reuptake, the substantially pure (1S, 2R) enantiomer of milnacipran hydrochloride, F2695, is especially useful in the preparation of a drug intended to treat a number of psychiatric disorders, while reducing the risk of suicidal behaviour and ideations.

Among these psychiatric disorders, disorders of the central nervous system as defined in <<The Diagnostic and Statistical Manual of Mental Disorders-IV (DSM-IV), 1995 American Psychiatric Association>> should be mentioned. By way of examples, but not limited to these, the following psychiatric disorders and conditions should be mentioned: depression, bi-polar disease, schizophrenia, generalized anxiety, morose and marasmic states, stress-related diseases, panic attacks, phobias, obsessive-compulsive disorders, behavioural disorders, depression of the immune system, fatigue and the associated pain syndromes, chronic fatigue syndrome, fibromyalgia, and other functional disorders, autism, disorders characterized by attention deficit due to general health status, attention disorders due to hyperactivity, eating disorders, neurotic bulimia, neurotic anorexia, obesity, psychotic disorders, apathy, migraine, pain, irritable bowel syndrome, cardiovascular diseases, neuro-degenerative diseases and the associated anxiety-depressive syndromes (Alzheimer's disease, Huntington's chorea, Parkinson's disease) urinary incontinence and drug addiction.

In the context of the present invention, the term "depression" is understood to refer to a constellation of symptoms having, on the one hand, a psychological aspect consisting of mood disorders with pessimism, moral suffering, thoughts of death or suicide, mental inhibition, and, on the other hand, a physical aspect of motor deficit, consisting in particular of a slowdown in motor activity, of appetite disturbances, of constipation, of sleep disturbances and of weight-control disturbances. Depression therefore corresponds to pathological psychological state combining a painful mood-alteration and a reduction in mental or motor activity. The term "depressive state" is understood to refer to a mental state characterised by a decline in neuropsychological tonicity, manifesting as lassitude, tendency to fatigue, discouragement, and tendency to pessimism sometimes accompanied by anxiety.

Hence, more specifically, the object of the present invention concerns the use of the substantially pure (1S, 2R) enantiomer of milnacipran hydrochloride, F2695, for the preparation of a drug intended to treat psychiatric disorders, and preferably depression, such as deep depression, resistant depression, psychotic depression, depression induced by treatments with interferon, depressive state, maniac-depressive syndrome, seasonal depressive state, depressive episodes related to general health status, depression related to mood altering substances, while reducing the risk of suicidal behaviour or ideation.

Alternatively, the present invention discloses a method for reducing the risk of suicidal behaviour or ideation in patients afflicted with psychiatric disorders, said method comprising administering to said patients an effective amount of the substantially pure (1S, 2R) enantiomer of milnacipran hydrochloride, F2695.

This method is preferentially applied to young patients suffering from psychiatric disorders as described previously.

As used herein, an "effective amount" of the substantially pure (1S, 2R) enantiomer of milnacipran, F2695, corresponds to doses from 0.01mg to 10mg/kg body weight per day in one or more intakes, preferably at doses from 0.05mg to 5mg/kg body weight per day in one or more intakes, and even more preferably at doses from 0.1mg to 1mg/kg body weight per day in one or more intakes In a particularly preferred manner, the effective amount of the substantially pure dextrogyral enantiomer of milnacipran, F2695 is at 25mg/day at the beginning of the treatment, and is progressively increased after 4-5 days to reach 75mg/day of F2695.

In the context of oral administration, the pharmaceutical compositions in accordance with the invention are advantageously administered in unit-dose or multiple-dose. These administration forms contain appropriate pharmaceutical media known by the man skill in the art.

As used herein, the term "unit dose" comprises solid pharmaceutical dosage forms of F2695 such as capsules, tablets, lozenges, pills, gel capsules, granulates, oral suspensions or solutions, or powders. Appropriate multiple-dose administration forms include in particular drinkable drops, emulsions and syrups.

Unit doses are preferably orally administrable capsules, each containing 25mg of F2695, or 50mg of F2695.

Release of the substantially pure (1S, 2R) enantiomer of milnacipran hydrochloride, F2695, may be delayed and/or controlled by using all kinds of controlled release galenic forms.

Hence, the present invention also relates to a pharmaceutical composition comprising the substantially pure (1S, 2R) enantiomer of milnacipran hydrochloride, F2695, for its use for treating patients suffering from psychiatric disorders, preferably from depression, while reducing the risk of suicidal behaviour or ideation in said patients.

As used herein, the term "pharmaceutical composition" comprises a solid mixture of F2695 with, optionally, pharmaceutically acceptable excipients, additives, adjuvants, carriers and/or diluents, such as water, monosaccharides, disaccharides, glycols, oils, or mixtures hereof, to which is optionally added colourings, aroma, preservatives, stabilisers, wetting agents, provided that they are compatible with the active ingredient F2695.

To prepare a pharmaceutical composition of the invention, an appropriate amount of F2695 is combined in an intimate admixture with a pharmaceutically acceptable carrier, which carrier can take a wide variety of forms depending on the form of preparation desired for administration orally, nasally, rectally, percutaneously or by parenteral injection, preferably for oral administration.

The substantially pure (1S, 2R) enantiomer of milnacipran hydrochloride, F2695, is advantageously administered to patients receiving simultaneously, separately, or staggered in time at least one other active compound in the treatment of above-mentioned disorders, in particular a psychotropic, for its use for limiting the risk of suicidality induced by such drug.

For the purpose of the invention, the phrases "while limiting the risk of suicidal behaviour and ideation" or "while limiting the risk of suicidality" is understood to mean the fact of preventing these risks from increasing significantly in a patient following administration of a drug, here the psychotropic.

The term "psychotropic" is understood to designate a substance of natural or artificial origin capable of modifying mental activity and whose action is essentially exerted on the central nervous system and the psychological state. Preferentially, said psychotropic is an anti-depressant. Preferably, said anti-depressant is chosen among Selective Serotonin Reuptake inhibitors (SSRI) and other actual anti-depressants.

Preferably, SSRI and other actual anti-depressants were chosen among bupoprion, citalopram, dulotexine, escitalopram, fluoxetine, fluovoxamine, mirtazapine, nefazadone, paroxetine, sertraline, and venlafaxine, or combinations thereof.

Preferentially, the object of the present invention also includes the use of the substantially pure (1S, 2R) dextrogyral enantiomer of milnacipran, in a drug intended to treat patients suffering from psychiatric disorders, especially depression, while limiting the risk of suicidal behaviour or ideations, **characterised in that** the drug contains also at least one active compound chosen among psychotropics, in particular anti-depressants, in particular SSRIs or other actual anti-depressants, as associated products for use simultaneously, separately or staggered in time.

Alternatively, the present invention discloses a product containing: a) said substantially pure (1S, 2R) dextrogyral enantiomer of milnacipran hydrochloride, F2695, and b) at least one other active substance selected among the SSRIs or other actual anti-depressants, as combined compounds for a simultaneous, separate, or staggered use for the treatment of psychiatric disorders, while limiting the risk of suicidal behaviour or ideations.

Said products may be included in a pharmaceutical composition, said composition comprising the substantially pure (1S, 2R) enantiomer of milnacipran hydrochloride, F2695 and one or several SSRIs or related anti-depressants, for its use for treating patients suffering from psychiatric disorders, in particular depression, while limiting the risk of suicidal behaviour or ideation in said patients. Preferably, SSRI or other actual anti-depressants were chosen among bupoprion, citalopram, dulotexine, escitalopram, fluoxetine, fluovoxamine, mirtazapine, nefazadone, paroxetine, sertraline, and venlafaxine, or combinations thereof. Patients are preferably chosen among children, adolescents, young adults (age 18-24) or patients that have suicidal behaviour or ideations. Preferably, this composition is useful for the treatment of patients suffering from depression, in particular deep depression, resistant depression, psychotic depression, depression induced by treatments with interferon, depressive state, maniac-depressive syndrome, seasonal depressive state, depressive episodes related to general health status, depression related to mood altering substances, while reducing the risk of suicidal behaviour or ideation.

An other aspect of the present invention is the substantially pure (1S, 2R) enantiomer of milnacipran hydrochloride, F2695, for its use for limiting the risk of suicidality induced by the treatment with SSRI or other actual anti-depressants, especially in the first phase of anti-depressant treatment.

Also encompassed by the invention is a method for limiting the risk of suicidal behaviour or ideations in patients treated by one or several SSRIs or other actual anti-depressants, said method comprising administering to said patients a composition comprising an effective amount of the substantially pure (1S, 2R) dextrogyral enantiomer of milnacipran.

Said SSRIs or other actual anti-depressants are chosen among bupoprion, citalopram, dulotexine, escitalopram, fluoxetine, fluovoxamine, mirtazapine, nefazadone, paroxetine, sertraline, and venlafaxine, or combinations thereof.

Finally, the present invention disclose a method for limiting the risk of suicidal behaviour in patients suffering from psychiatric disorders, comprising administering: a) said substantially pure dextrogyral enantiomer of milnacipran, F2695, and b) at least one other active substance selected among the SSRIs or other actual anti-depressants, wherein said products a) and b) are administered simultaneously, separately, or staggered in time.

This method is intended to be applied to patients who may be likely to develop suicidal behaviour or ideations during or following the treatment with anti-depressants such as SSRI or other actual anti-depressants, especially children, adolescents or young adults (age 18-24), or patients that have already suicidal behaviour or ideations. This method is preferentially applied to patients suffering from psychiatric disorders as described previously.

### EXPERIMENTAL PROCEDURES

### Methodology

This was a multi-center, randomised, double-blinded, placebo-controlled 10 weeks study, assessing the efficacy and safety of F2695 flexible safety adapted dose in the treatment of patients with Major Depressive Disorders (MDD).

This study enrolled 563 patients who met the DSM-IV criteria for major depression with depression severity at the inclusion time assessed by HAM-D 17 score >22 .

Patients were randomised to receive placebo or a flexible safety adapted dose by increasing progressively the administered doses using slow release capsules containing 25 or 50mg of active drug (281 patients for placebo and 282 patients for F2695).

Placebo as well as F2695 were administered once a day as slow release capsules.

Slow release capsules were constituted of a plurality of minigranules each containing an active minisphere comprising a sucrose core containing F2695 as well as PVP as a binding agent; each minigranule being coated with a film of ethyl cellulose as described in EP 939 626. Placebo was constituted of the same slow release capsules of minigranules containing minispheres constituted only of a sucrose core.

The doses were administered in a dose escalation regimen during 10 days for initial titration.

All patients were scheduled to receive a total of 10 weeks of F2695 or placebo followed by 7 days down titration over 10 days follow-up period.

### Assessments

Safety of F2695 was assessed by analysing:
- frequency and intensity of adverse events as classified in the ICH guidelines (any adverse change from the subject's baseline condition, *i*.*e*. any subjective signs and symptoms, or change in a concomitant disease present at the selection visit, either considered or not related to the treatment)
- suicidal risk evaluated by the MINI version 0.5 (modified Mini International Neuropsychiatric Interview)

Efficiency of F2695 was assessed by:
- MADRS Total score: change from baseline MMRM analysis (Full analysis data set)
- MADRS Total score: change from baseline MMRM analysis (Per Protocol data set)
- Responders and remitted patients compared to placebo group at Day 70 (end of the treatment period)

### Results from Safety

Following Table 1 summarizes the safety results of this study:

| | **Placebo** **n=281** | | **F2695** **n=282** | | **Total** **n=563** | |
|---|---|---|---|---|---|---|
| **Number of withdrawn patients** | 70 | ( 24.9%) | 57 | ( 20.2%) | 127 | ( 22.6%) |
| **Significant Suicidal Risk** | 6 | ( 2.1%) | 1 | ( 0.4%) | 7 | ( 1.2%) |
| **Serious Adverse Event/Non Serious Adverse Event** | 16 | ( 5.7%) | 26 | ( 9.2%) | 42 | ( 7.5%) |
| **Therapeutic Failure** | 40 | ( 14.2%) | 22 | ( 7.8%) | 62 | ( 11.0%) |
| **Worsening of MDD** | 17 | ( 6.0 %) | 11 | ( 3.9%) | 28 | ( 5.0%) |
| **Insufficient response** | 33 | (11.7%) | 16 | ( 5.7%) | 49 | ( 8.7%) |
| **Patient's decision - Consent withdrawal** | 37 | ( 13.2%) | 29 | ( 10.3%) | 66 | ( 11.7%) |
| **Patient lost to follow up** | - | | 1 | ( 0.4%) | 1 | ( 0.2%) |
| Other reason | 10 | ( 3.6%) | 8 | ( 2.8%) | 18 | ( 3.2%) |

### Data Analysis

The above-mentioned results highlight both the anti-depressant effect, and favourable effect on suicidal risk of F2695 under the study conditions.

Upon placebo treatment, 6 patients (2.1 %) present significant Suicidal Risk, versus 1 patient (0.4%) for the group treated with F2695. One could calculate in this case an odds ratio (OR) of 0.1637, with a confidence interval of [0,0196 ; 1,3686] (logits method).

The weakness of this odds ratio obtained in a small population indicates that the Suicidal Risk is significantly reduced by F2695 treatment in patients suffering from depression.

As it is known that the events rates of suicidal acts or ideation are highest in the major depressive disorder (MDD) indication compared with other psychiatric disorders, this result can be compared with results obtained in several trials with patients suffering from psychiatric disorders (among them MDD) as synthesized in the document: Pharmacovigilance Working Party on Antidepressants and suicidal thoughts and Behaviour, January 2008, www.hma.eu/uploads/media/PAR_suicidal_thoughts_in_antidepressants.pdf - that tends to indicate that other anti-depressant drugs (such as bupropion, citalopram, escitalopram and fluvoxamine) may increase suicidal behaviour and ideation because their odds ratio versus placebo were superior to 1.

For example, OR (bupropion/placebo) = 1,41, OR (citalopram) = 2,21, OR (escitalopram) = 1,57, OR (fluvoxamine) = 1,37.

Hence, whereas other anti-depressant drugs tend to increase the suicidal risks when depressed patients are treated, F2695 induces a limitation and even a reduction of this risk.

## Claims

1. The substantially pure (1S, 2R) enantiomer of milnacipran hydrochloride for its use for treating patients suffering from psychiatric disorders, while reducing the risk of suicidal behaviour or ideation in said patients.

2. Use of the substantially pure (1S, 2R) enantiomer of milnacipran hydrochloride for the preparation of a drug intended to treat patients suffering from psychiatric disorders, while reducing the risk of suicidal behaviour or ideation in said patients.

3. The use according to claim 1 and 2, wherein said patients are chosen among children, adolescents and young adults of ages 18-24.

4. The use according to claim 1 to 3, wherein said patients have suicidal behaviour or ideation.

5. The use according to claim 1 to 4, wherein said psychiatric disorders are selected from depression, bi-polar disease, schizophrenia, generalized anxiety, morose and marasmic states, stress-related diseases, panic attacks, phobias, obsessive-compulsive disorders, behavioural disorders, depression of the Immune system, fatigue and the associated pain syndromes, chronic fatigue syndrome, fibromyalgia, and other functional disorders, autism, disorders **characterized by** attention deficit due to general health status, attention disorders due to hyperactivity, eating disorders, neurotic bulimia, neurotic anorexia, obesity, psychotic disorders, apathy, migraine, pain, irritable bowel syndrome, cardiovascular diseases, neuro-degenerative diseases and the associated anxiety-depressive syndromes (Alzheimer's disease, Huntington's chorea, Parkinson's disease) urinary incontinence and drug addiction.

6. The use according to claim 1 to 5, wherein said patients suffer from depression.

7. The use according to claim 6, wherein depression is selected from deep depression, resistant depression, psychotic depression, depression induced by treatments with interferon, depressive state, maniac-depressive syndrome, seasonal depressive state, depressive episodes related to general health status, depression related to mood altering substances.

8. The use according to claim 1 to 7, wherein said patients are treated with one or several selective serotonine reuptake inhibitors (SSRIs) or other actual anti-depressants.

9. The use according to claim 8, wherein said SSRIs or other actual anti-depressants are chosen among bupoprion, citalopram, dulotexine, escitalopram, fluoxetine, fluovoxamine, mirtazapine, nefazadone, paroxetine, sertraline, and venlafaxine, or combinations thereof.

10. Product containing: a) said substantially pure (1S, 2R) enantiomer of milnacipran hydrochloride, and b) at least one other active substance selected among the SSRIs or other actual anti-depressants, as combined compounds for a simultaneous, separate, or staggered use for the treatment of psychiatric disorders, while limiting the risk of suicidal behaviour or ideations.

11. The substantially pure (1S, 2R) enantiomer of milnacipran hydrochloride for its use for limiting the risk of suicidal behaviour or ideation induced by the treatment with one or several SSRIs or other actual anti-depressants.

12. A pharmaceutical composition comprising the substantially pure (1S, 2R) enantiomer of milnacipran hydrochloride and one or several SSRIs or other actual anti-depressants, for its use for treating patients suffering from psychiatric disorders, while limiting the risk of suicidal behaviour or ideations in said patients.
